(19) Europäisches Patentamt / European Patent Office / Office européen des brevets

(11) **EP 4 289 383 A1**

(12) **EUROPEAN PATENT APPLICATION**

(43) Date of publication:
**13.12.2023   Bulletin 2023/50**

(21) Application number: **22178223.8**

(22) Date of filing: **09.06.2022**

(51) International Patent Classification (IPC):
**A61B 34/30** (2016.01)     **A61B 34/00** (2016.01)
**A61B 34/20** (2016.01)     **A61B 90/00** (2016.01)

(52) Cooperative Patent Classification (CPC):
**A61B 34/30; A61B 34/76;** A61B 2034/2055;
A61B 2034/2059; A61B 2034/304; A61B 2090/064;
A61B 2090/066

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Medos International Sarl**
  **2400 Le Locle (CH)**
• **Rheinisch-Westfälische Technische**
  **Hochschule Aachen**
  **52062 Aachen (DE)**

(72) Inventors:
• **RADERMACHER, Klaus**
  **52074 AACHEN (DE)**
• **DE LA FUENTE KLEIN, Matías**
  **52074 AACHEN (DE)**
• **THEISGEN, Lukas**
  **52074 AACHEN (DE)**
• **VOSSEL, Manuel**
  **52074 AACHEN (DE)**

(74) Representative: **Regimbeau**
  **20, rue de Chazelles**
  **75847 Paris Cedex 17 (FR)**

(54) **ROBOTIC SURGICAL SYSTEM**

(57)     The present disclosure relates to a robotic surgical system, comprising:
- a robot (1A-1C) holding a surgical tool (3A-3C), the robot comprising a base (10), a flange (12) coupled to the surgical tool and an actuation unit (11) configured to move the flange (12) relative to the base (10);
- a robotic carrier arm (2) comprising at least one joint (22A-22G), the carrier arm comprising a flange (23) removably connected to the base (10) of the robot (1A-1C), the carrier arm being configured to maintain position of the robot base (10) in a fixed joint configuration during operation of the robot (1A-1C);
- a localization unit configured to determine in real time position of the surgical tool;
- a control unit configured to:
- determine the position of the surgical tool (3A-3C) based on data received from the localization unit;
- carry out a control loop for dynamically compensating elastic deformation of the carrier arm, the control loop comprising, based on a target position of the surgical tool, on a current joint position of the actuation unit, and on the position of the surgical tool, determining at least one command of the actuation unit to achieve the target position.

FIGURE 1

## Description

## TECHNICAL FIELD

The present disclosure relates to a robotic surgical system.

## TECHNICAL BACKGROUND

Surgical robotic systems are increasingly used to assist surgeons in practicing surgical interventions that require a high degree of accuracy.

Various types of surgical robotic systems have been proposed.

Large and stiff robotic arms, comparable to industrial robots, have been developed to execute surgical tasks. However, such robotic arms are quite cumbersome and their use in an operating room already comprising various systems may raise problems in terms of spatial arrangement. In addition, such robotic arms usually have a large working space, which may create safety problems, due to the risk of collision with the patient, the medical staff and/or other devices present in the operating room.

On the other hand, small robots to be mounted directly to the patient's bone or to be handheld have been proposed. These smaller robots, however, have a limited working space which can require frequent repositioning of the robot in order to achieve a surgical plan.

## SUMMARY OF THE DISCLOSURE

A goal of the present disclosure is to propose a robotic surgical system that allows saving space in the operating room while ensuring accuracy in the positioning of the surgical tool.

Accordingly, the robotic surgical system comprises:

- a robot holding a surgical tool, the robot comprising a base, a flange coupled to the surgical tool and an actuation unit configured to move the flange relative to the base;
- a robotic carrier arm comprising at least one joint, the carrier arm comprising a flange removably connected to the base of the robot, the carrier arm being configured to maintain position of the robot base in a fixed joint configuration during operation of the robot;
- a localization unit configured to determine in real time position of the surgical tool;
- a control unit configured to:

  - determine the position of the surgical tool based on data received from the localization unit;
  - carry out a control loop for dynamically compensating elastic deformation of the carrier arm, the control loop comprising, based on a target position of the surgical tool, on a current joint position of the actuation unit, and on the position of the surgical tool, determining at least one command of the actuation unit to achieve the target position.

The robotic surgical system thus combines a robotic carrier arm, that has a large working space, and a tooling robot with a small working space, which is carried by the carrier arm. The robotic carrier arm may advantageously be lightweight so as to provide a minimized bulkiness, but it thus has a reduced stiffness which may generate elastic deformation of the arm, depending in particular on the joint configuration.

The robotic carrier arm allows performing coarse pre-positioning of the tooling robot to the surgical field of the planned procedure, and then locks the flange of the robotic carrier arm in a fixed position. While the flange of the robotic arm (and thus the base of the tooling robot) is in the fixed position, the tooling robot adjusts the position and orientation of the surgical tool and may further compensate for elastic deformation of the robotic carrier arm and the tooling robot and, if appropriate, relative motion of the patient.

In some embodiments, dynamically compensating the elastic deformation of the carrier arm further comprises determining the at least one command of the actuation unit based on a kinematic model of the actuation unit.

In some embodiments, dynamically compensating the elastic deformation of the carrier arm further comprises determining the at least one command of the actuation unit based on a dynamic model of the robotic carrier arm.

Preferably, the robotic carrier arm has six or seven degrees of freedom.

In some embodiments, the robotic carrier arm has a serial architecture.

In some embodiments, the control unit is further configured to:

- determine a current joint configuration of the robotic carrier arm;
- based on a model of the robotic carrier arm, determine at least the lowest resonant frequency of the carrier arm in the current joint configuration;

wherein the control loop comprises applying a filter configured to dampen movement of the actuation unit of the robot at least at the lowest resonant frequency of the robotic carrier arm.

The control loop may further comprise:

- determining a position of the surgical tool in a first coordinate system;
- determining a position of the patient in a second coordinate system;
- based on a current joint configuration of the actuation unit and a kinematic model of the actuation unit, determining a current position of the base in a third coordinate system;

- based on the position of the surgical tool in the first coordinate system and on the position of the base in the third coordinate system, determining the current position of the base in the first coordinate system;
- filtering at least the lowest resonance frequency of the carrier arm out of the position of the base in the first coordinate system;
- based on the position of the patient in the second coordinate system and on the filtered position of the base in the first coordinate system, determining the position of the base in a fourth coordinate system;
- based on a target position of the surgical tool in the fourth coordinate system, determining a target position of the surgical tool in a fifth coordinate system;
- implementing the kinematic model of the actuation unit to determine a command of the actuation unit to achieve the target position of the surgical tool.

In some embodiments, each joint of the robotic carrier arm comprises an encoder and the control unit is configured to determine the current joint configuration of the carrier arm based on data provided by each encoder.

The robotic carrier arm may advantageously be configured to be moved manually by a user to bring the robot to a target position.

The robotic surgical system may further comprise at least one sensor configured to determine an intention of the user when moving the robotic carrier arm and the control unit may be configured to command at least one motor of the robotic carrier arm based on measurements provided by said at least one sensor to reach a target pose of the flange of the robotic carrier arm.

Advantageously, the surgical tool may be configured to be moved manually by a user.

The surgical robotic system may further comprise at least one sensor configured to determine an intention of the user when moving the surgical tool, the control unit being configured to command the actuation unit of the robot based on measurements provided by said sensor during operation of the surgical tool by the user.

In addition, the control unit may be configured to provide haptic guidance to the user when moving the robotic carrier arm or the surgical tool.

In some embodiments, the localization unit comprises a camera and at least one array of markers detectable by the camera is fixed to the surgical tool to determine in real time the position of the surgical tool.

In some embodiments, the surgical tool is a cutting tool, in particular a saw, a drilling tool, a burring tool, a reaming tool or a milling tool, a burning tool, or an implant holder.

## BRIEF DESCRIPTION OF THE FIGURES

Further features and advantages of the system will be apparent from the following description, based on the appended drawings, wherein:

- FIG. 1 is a view of the carrier arm and three different tooling robots adapted to be coupled to the flange of the carrier arm;
- FIGS. 2A to 2C are views showing three different joint configurations of the robotic carrier arm;
- FIG. 3 is a diagram representing a control loop implemented by the control unit to compensate for elastic deformation of the surgical robotic system and movements of the patent.

## DETAILED DESCRIPTION OF EMBODIMENTS

The robotic surgical system combines a robotic carrier arm, that has a large working space, and a robot with a small working space, which is carried by the carrier arm and holds a surgical tool. The robot holding the surgical tool is also called "tooling robot" in the following description.

The robotic surgical system is intended to be used in a pre-planned surgical procedure, for example in orthopedic surgery, cranial surgery, neurosurgery, otorhinolaryngology, etc.

As will be explained in more detail below, the robotic carrier arm is used for coarse pre-positioning of the robot holding the surgical tool. Then, the robotic carrier arm maintains the position of the robot holding the surgical tool during implementation of the surgical task according to the surgical plan.

### Tooling robot

The tooling robot comprises a base, a flange adapted to releasably receive the surgical tool and an actuation unit configured to move the flange (and thus the surgical tool attached thereto) relative to the base.

The base is attached to a flange of the robotic carrier arm.

The actuation unit may have a serial or a parallel architecture, or a combination of serial and parallel architectures.

The tooling robot is preferably lightweight and highly reactive so as to be able to quickly adjust the position of the tool and also compensate for elastic deformation of the robotic system.

Such a tooling robot typically has a small working space. Depending on the tool type, the actuation unit may have one or more degrees of freedom.

### Tool

The tool may be an off-the-shelf surgical tool which would advantageously not need to be specifically adapted for use with robotic surgical systems.

The flange of the tooling robot may comprise an interface adapted to couple to various kinds of tools.

In particular, a surgical intervention may require use of several tools, successively. A first tool may thus be coupled to the flange of the tooling robot, used to implement the required surgical step, and then removed and replaced by another tool.

In some embodiments, the tool may be a tool that affects the patient's tissues. For example, the tool may be a powered cutting tool, such as a saw, a drilling tool, a burring tool, a reaming tool or a milling tool. In other examples, the tool may be a burning tool.

In other embodiments, the tool may not interact with the patient's tissue. For example, the tool may be a guide tube that allows a user to drill through the tube at a set angle and location. Alternatively, the tool may be an implant holder carrying an implant to be placed in the patient's body.

Of course, the provided examples are not limitative.

Robotic carrier arm

The robotic carrier arm comprises a base, an arm having a proximal end extending from the base and comprising at least one joint, and a flange extending at a distal end of the arm.

Preferably, the arm has an anthropomorphic design with a serial architecture formed of a plurality of segments and comprising motorized joints (e.g., servo motors) connecting two successive segments.

In some embodiments, each joint comprises an encoder. In this way, it is possible to determine the joint configuration of the robotic carrier arm at any time thanks to measurement data (e.g. angular position of the motor of each joint) provided by the encoders.

The base of the robotic carrier arm may be mounted on a movable cart so as to bring the robotic carrier arm in the vicinity of the operating table during the surgical intervention. Alternatively, the base of the robotic carrier arm may be directly mounted onto rails on the operating table.

The flange of the robotic carrier arm is configured for releasable attachment of the base of the robot carrying the tool. In particular, depending on the surgical intervention, various tooling robots may be interchangeably attached to the flange of the robotic carrier arm. In particular, if the surgical intervention comprises at least two steps requiring different tools, two or more different tooling robots may be successively attached to the flange of the robotic carrier arm.

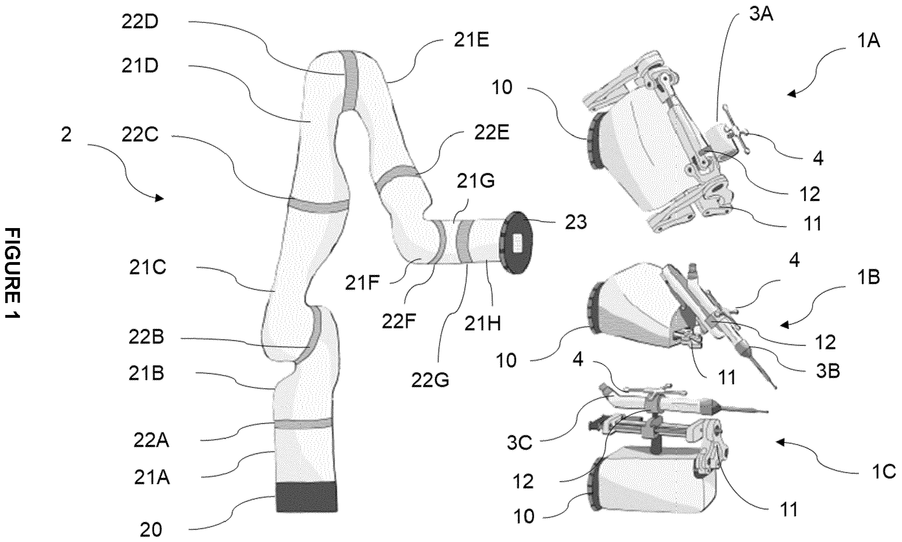

FIG. 1 is a schematic view of the robotic carrier arm and three different tooling robots adapted to be coupled to the flange of the carrier arm.

From top to bottom, the first tooling robot 1A holds a guide 3A for drilling tasks, the second tooling robot 1B holds a milling tool 3B for laminectomy, and the third tooling robot 1C holds a milling tool 3C for uni knee arthroplasty (UKA). The second tooling robot has a rather cubic workspace whereas the third tooling robot has a rather long workspace.

Each tooling robot 1A, 1B, 1C comprises a base 10, a flange 12 coupled to the respective tool 3A, 3B, 3C and a motorized actuation unit 11 connecting the base 10 to the flange 12. It can also be seen a tracker 4 comprising an array of markers attached to each tool 3A, 3B, 3C. Such a tracker can be tracked by a camera of an optical localization system.

The robotic carrier arm 2 comprises a base 20, a flange 23 and a plurality of segments 21A-21H connected by motorized joints 22A-22G.

The flange 23 of the robotic carrier arm is configured to be rigidly coupled to the base 10 of any one of the tooling robots 1A-1C.

Of course, the shape and architecture of the robotic carrier arm and the tooling robots is given as an illustration and is not intended to be limitative.

The robotic carrier arm comprises at least one degree of freedom.

In preferred embodiments, the robotic carrier arm is an off-the-shelf 6- or 7-degrees of freedom robotic arm. Such a robotic carrier arm is, for example, commercially available from KUKA, KINOVA, FRANKA EMIKA, UNIVERSAL ROBOTS or ABB ...

Preferably, the robotic carrier arm is of a thin and lightweight type, with reduced motor torques. Such a lightweight arm typically comprises, at each joint, a motor with a gearbox, a torque sensor and electronics, and the joints are connected by carbon fiber and/or aluminum shells forming the segments.

An advantage of such a lightweight, slender carrier arm is that its bulkiness is minimized, thereby saving space in the operating room. In addition, the risk of hurting the patient or the medical staff during operation of the robotic carrier arm is reduced as compared to stiff robotic arms.

The above-mentioned off-the-shelf 6- or 7-degrees of freedom robotic arms belong to this category of lightweight robotic arms.

A counterpart of such a lightweight architecture is that the robotic carrier arm is not very stiff and that it may elastically deform in particular due to the weight of the robot holding the tool and due to forces exerted by the tooling robot during operation of the tool. The thinner and lighter the robotic carrier arm is, the more elastic it becomes and even small forces lead to a deflection of the robotic carrier arm. For example, the stiffness of the robotic carrier arm can be of the order of 5 N.mm$^{-1}$.

However, the highly dynamic robot holding the tool allows compensating for such elastic deformation, without moving the robotic carrier arm. In this way, potentially large autonomous movements of the robotic carrier arm are avoided and the safety of the system is increased.

It is to be noted that the deformation of the robotic carrier arm may depend on the joint configuration (i.e. the position of the segments of the robotic carrier arm relative to each other). In particular, depending on whether the robotic arm is in an extended or stretched configuration or in a compact one, the resonant frequency of the robotic carrier arm may vary (said resonant frequency being lower when the arm is in an extended configuration). For example, the lowest resonant frequency of the robotic carrier arm can be of the order of 5 or 10 Hz.

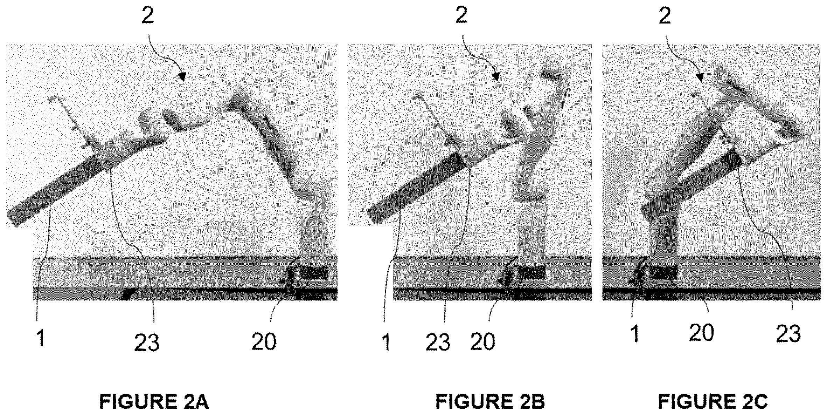

FIGS. 2A to 2C illustrate respectively the robotic carrier arm 2 in a stretched configuration, in a medium configuration and in a narrow, compact configuration. In these figures, the tooling robot attached to the flange 23 of the robotic carrier arm has been represented schematically by a bar 1. The lowest resonant frequency increases from the stretched configuration to the narrow configuration.

In order not to stimulate the robotic carrier arm at its resonance frequency during the dynamic compensation (which would cause oscillation of the robotic carried arm and thus instability of the system), said resonance frequency may be eliminated by an appropriate filter in the control loop of the tooling robot.

Localization unit

The localization unit is configured to determine in real time the position of the tool and, if appropriate, the position of the patient. To that end, the tool and, if appropriate, the patient, have a tracker rigidly attached thereto. The localization unit tracks the position of each tracker in real time, at a high speed (for example, about 335 Hz) and with low latency (for example about 5ms).

Various localization techniques are known and can be used in the present robotic surgical system. For example, an optical localization unit comprises a camera and each tracker is an array of markers detectable by the camera. In other examples, electromagnetic localization involves electromagnetic emitters and sensors.

Depending on the surgical intervention, the anatomical structure may be fixed to the operating table. This is for example the case in cranial surgery, wherein the patient's head is firmly clamped. In such case, it may not be necessary to attach a tracker to the patient, the localization unit being supposed to remain in a fixed known position relative to the patient's head.

In other surgical interventions, in particular in orthopedic surgery, the anatomical structure may be maintained by a holding system but may be subject to small movements, especially due to breathing. In such case, at least one tracker is attached to the patient's body in order to follow patient's movements.

Control unit

The robotic surgical system comprises a control unit configured to control operation of both the robotic carrier arm and the robot holding the tool.

The control unit typically comprises at least one processor configured to implement computer programs or algorithms, at least one memory, and input/output connectors.

The control unit receives a surgical plan which may be defined separately from the present robotic surgical system. The surgical plan may be determined by a surgeon based on medical image of the patient's anatomy. The surgical plan may typically comprise a surface or a volume of an anatomical structure to be treated, an entry point and/or an entry axis for the tool, etc.

The control unit receives data from the localization unit and is thus configured to determine in real time the positions of the surgical tool and the patient.

The control unit also implements a compensation control loop that will be described in more detail below, in order to compensate for elastic deformation of the lightweight robotic carrier arm.

Further, both the robotic carrier arm and the tooling robot may be moved by the user using hands-on control. The control unit may thus be configured to implement an admittance control loop.

Use of the robotic surgical system

The robotic carrier arm is advantageously moved by the user thanks to a hands-on synergistic control in order to pre-position the robot holding the tool. In this way, the user takes into account obstacles present in the operating room and the risk of harming the patient during a large amplitude autonomous displacement of the robotic carrier arm can be avoided.

Admittance control is particularly suited for an elastic robotic arm. In admittance control, the forces and torques applied by the user onto the robotic arm are measured and, from those, the control unit calculates an intended pose of the flange of the robotic carrier arm, which is then realized by the motors of the arm.

Preferably, haptic guidance is also provided to guide the user toward the target pose of the flange. The more accurate the pre-positioning can be performed, the smaller the workspace of the robot holding the tool can be designed.

The admittance control algorithm may be integrated in the off-the-shelf robotic carrier arm and thus will not be described in detail in the present text.

The admittance control compensates for any friction in the joints and automatically compensates for the own weight of the robotic carrier arm and the weight of the attached tooling robot. It also allows additional forces and torques to be set via an application programming interface, which are then applied at the flange of the robotic carrier arm. These settings are used to implement the haptic guidance with so-called virtual fixtures.

Thus, virtual walls can be created with sufficient rigidity to impede movement of the robotic carrier arm in forbidden regions.

Once the tooling robot is in the target position, in which the workspace of the tooling robot allows treating the anatomical structure of the patient with the tool, the robotic carrier arm holds the position of the flange of the robotic carrier arm (and thus of the base of the tooling robot).

Then, the user can use the tool to carry out the planned intervention thanks to hands-on control of the tooling robot. To that end, the control unit may carry out an admittance control loop. Alternatively, the tooling robot may autonomously operate the surgical tool to achieve the surgical plan.

In both cases, the control unit implements a control loop to control the actuation unit of the tooling robot in order to dynamically compensate for elastic deformation of the carrier arm and the actuation unit of the tooling robot, while avoiding exciting the robotic carrier arm by the tooling robot at least at the lowest resonant frequency of the robotic carrier arm.

Admittance control

Admittance control is known per se and will not been described in detail in the present text. One may refer in particular to publications [1]-[3].

In order to implement admittance control, the surgical robotic system includes at least one sensor configured to determine an intention of the user when moving the robotic carrier arm and/or the tooling robot.

For example, a force torque sensor can be used to measure the force applied by the user during the steering. Such a force torque sensor is advantageously arranged between the flange of the robotic carrier arm and the base of the tooling robot, but other locations may also be used.

Alternatively, the intention of the user can be determined based on forces and/or torques measured at each joint of the robotic carrier arm. Said measurement can be direct (via force and/or torque sensors) or indirect (for example by measuring electrical current provided to the motors of the robotic carrier arm).

During the surgical task, the control includes haptic guidance in order to ensure that the tool is only moved within the region to be worked, without affecting sensitive tissue.

Thus, the control may include virtual fixtures based on an image-based surgical plan.

Various types of virtual fixtures may be used. Since such virtual fixtures are known, they will not be described in detail in the present text. One may refer in particular to publication [4].

Compensation control loop

During surgery assisted by the surgical robotic system, the robotic carrier arm holds its position and the tooling robot holding the surgical tool is responsible for the task itself.

The control loop comprises, based on a target position of the surgical tool, on a current joint position of the actuation unit, on the position of the surgical tool, determining at least one command of the actuation unit to achieve the target position of the tool.

The control loop is designed to compensate for elastic deformations of the robotic carrier arm and the robot holding the surgical tool. In addition, if the anatomical structure to be treated is allowed to move (for example due to patient's breathing), the control loop also compensates for motion of the anatomical structure.

Preferably, internal and, if any, external motions are compensated for separately. In the present text, internal motion means that the tooling robot is deflected due to external forces, i.e. the tool tracker moves unintentionally, or that the kinematic model of the tooling robot is inaccurate and the actual motion of the tool does not equal the intended motion, which can also be noticed by the motion of the tool tracker monitored by the localization unit. External motion means that the patient's anatomical structure moves, which is measured by the localization unit (e.g. the optical tracking camera monitoring the markers array attached to the patient).

Both phenomena can be corrected using the same control loop, but tuning of the control can be done individually. Indeed, the patient's movement (if any) is not influenced by the tooling robot's movement and thus is not fed back into the control loop and cannot lead to an unstable behavior. Therefore, this part of the compensation can be performed at higher frequencies than compensation for any tool deflection. As noted above, if the anatomical structure of the patient is clamped and cannot move, this part of the compensation is not necessary and may thus not be implemented.

A kinematic model of the actuation unit is recorded in a memory of the control unit. Said kinematic model is designed to determine, based on the position of each joint of the actuation unit, the position of the flange of the tooling robot relative to the base of the tooling robot (forward kinematic model). Conversely, the kinematic model allows determining, from a position of the flange of the tooling robot relative to the base of the tooling robot, a position of all joints of the actuation unit allowing placing the flange of the tooling robot in said position relative to the base (reverse kinematic model).

The position of the surgical tool is determined in a first coordinate system. Since the tool is tracked by the localization unit, the first coordinate system may be the coordinate system of the localization unit.

The position of the patient is determined in a second coordinate system. The second coordinate system may be identical to the first coordinate system or different from the first coordinate system. For example, especially if the anatomical structure of the patient is able to move (for example due to patient's breathing), the localization unit tracks the position of a tracker attached to the anatomical structure and the second coordinate system may be the coordinate system of the localization unit. In other situations, especially if the anatomical structure of the patient is not able to move during the surgical intervention (for example due to the fact that it is clamped in a fixed position relative to the operative table), the position of the anatomical structure may be determined only once, at the beginning of the surgical intervention.

Based on a current joint position of the actuation unit and a kinematic model of the actuation unit, the control unit determines a current position of the base of the tooling robot in a third coordinate system. For example, the third coordinate system may be the coordinate system of the tool.

Based on the position of the surgical tool in the first coordinate system and on the position of the base of the tooling robot in the third coordinate system, the control unit determines the current position of the base in the first coordinate system.

The control unit determines a filter configured to filter out at least the lowest resonant frequency of the robotic carrier arm. Since the lowest resonant frequency of the carrier arm varies depending on the joint configuration of the carrier arm, the determination of the resonant frequency (and thus of the filter) can be done dynamically at each sequence of the control loop. Alternatively, since the joint configuration of the robotic carrier arm is substantially fixed after the coarse pre-positioning is performed, the determination of the resonant frequency can be done only once after each pre-positioning step.

The filter may also be configured to eliminate higher resonant frequencies in addition to the lowest one.

To that end, the control unit determines the current joint configuration of the robotic carrier arm. Said determination may be made based on data provided by the encoders of the motors of the robotic carrier arm. Alternatively, the current joint configuration may be determined using the localization unit (tracking trackers attached to one or several parts of the robotic carrier arm), or using any other suitable means.

A dynamic model of the robotic carrier arm may be recorded in the memory of the control unit. Said dynamic model does not need to be complete, but allows estimating at least the lowest resonant frequency (and possibly additional higher resonant frequencies) of the robotic carrier arm. For example, the robotic carrier arm can be modelled as a damped harmonic oscillator.

The filter can be of any known type allowing dampening movement of the actuation unit of the robot. For example, the filter can be:

- a low-pass filter: the Laplace transfer functions for first- and third-order low-pass filters are respectively:

$$H(s) = \frac{1}{1 + \dfrac{s}{\omega_c}}$$

and

$$H(s) = \frac{1}{\left(1 + \dfrac{s}{\omega_c}\right)\left(1 + \dfrac{s}{\omega_c} + \dfrac{s^2}{\omega_c^2}\right)}$$

with $\omega_c$ denoting the -3dB cut-off angular frequency;

- a band-stop filter, which has the advantage of reaching a very high level of attenuation at its zero frequency. This zero frequency can be set to the estimated resonant frequency of the robotic carrier arm. The Laplace transfer function for the band-stop filter is:

$$H(s) = \frac{s^2 + \omega_z^2}{s^2 + \dfrac{\omega_p}{Q}s + \omega_p^2}$$

with $\omega_z$ denoting the zero angular frequency determining the completely filtered-out frequency, $\omega_p$ denoting the pole angular frequency, which can be set lower than $\omega_z$ to obtain low-pass behavior, and Q determining the bandwidth of the filter;

- a lead-lag filter, in which the Laplace function comprises additional zeroes in order not to add an increasing amount of attenuation of the system, which would slow down the step response. The Laplace function of a third-order lead-lag filter is:

$$H(s) = \frac{\left(1 + \dfrac{s}{\omega_z}\right)\left(1 + \dfrac{s}{\omega_z} + \dfrac{s^2}{\omega_z^2}\right)}{\left(1 + \dfrac{s}{\omega_p}\right)\left(1 + \dfrac{s}{\omega_p} + \dfrac{s^2}{\omega_p^2}\right)}$$

with $\omega_p$ denoting the -3dB pole angular frequency and $\omega_z$ denoting the zero angular frequency with an attenuation 3dB less than maximum. The maximum attenuation at high frequencies can be determined by varying the distance between the pole and the zero, with an attenuation of -60 dB per decade of pole-to-zero distance;
  or
- a combination of such filters.

The damping at the lowest resonant frequency (and for any higher resonant frequency) of the carrier arm may depend on the weight and architecture of the robotic carrier arm, the tooling robot and the surgical tool.

Based on the current joint configuration of the robotic carrier arm, the control unit determines at least the lowest resonant frequency of the robotic carrier arm and computes the filter accordingly. Then, the control unit applies this filter to the position of the base of the tooling robot in the first coordinate system to filter out at least the lowest resonant frequency of the carrier arm.

Based on the position of the patient in the second coordinate system and on the filtered position of the base in the first coordinate system, the control unit determines the position of the base of the tooling robot in a fourth coordinate system; for example, said fourth coordinate system may be the coordinate system of the patient.

Based on a target position of the surgical tool (which is known from the surgical plan) in the fourth coordinate system, the control unit determines a target position of the surgical tool in a fifth coordinate system; for example, said fifth coordinate system can be the coordinate system of the base of the tooling robot.

Then, the control unit implements the kinematic model of the actuation unit to determine a command of the actuation unit to achieve the target position of the surgical tool.

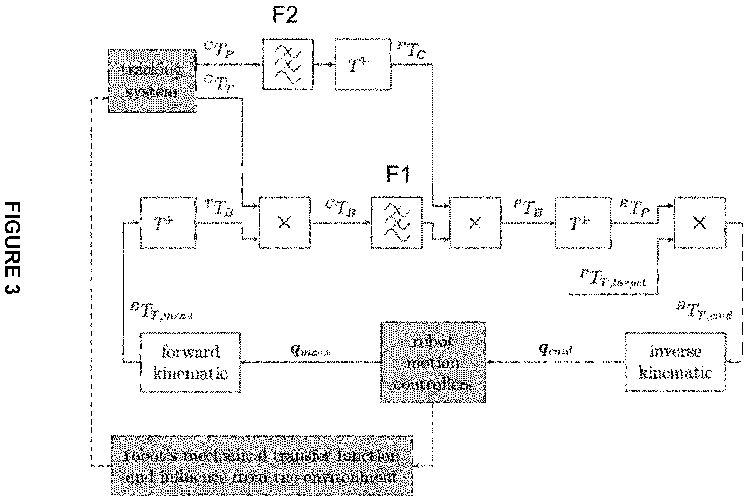

A flowchart of an embodiment of the dynamic compensation control loop applied to the actuation unit of the tooling robot to adjust the position of the tool is shown in FIG. 3.

All poses are given as homogeneous transformation matrix $^jT_i$ which transforms a vector from one coordinate system (noted coordinate system i) into another coordinate system (noted coordinate system j). In FIG. 3, T designates the coordinate system of the tool, B designates the coordinate system of the base of the tooling robot, P designates the coordinate system of the patient and C designates the coordinate system of the localization unit, which may be a tracking camera.

The localization unit measures the pose of the tool tracker and, if any, the patient tracker, said pose(s) being determined in the coordinate system C of the localization unit.

From the motion controllers, the current joint position of the actuation unit of the robot, $q_{meas}$, is obtained.

From the measured joint position, and the measured tool pose by the localization unit, the pose of the base B of the tooling robot in the coordinate system of the localization unit is computed as $^CT_B$. Due to elastic deformations of the robot or inaccuracies in the kinematic model, this may not be exactly the actual pose of the base of the robot.

A filter F1 (see description above) is implemented to add damping to the control loop at least at the lowest resonant frequency of the robotic carrier arm and thereby make it stable. The filter also reduces the noise from the localization unit.

The tracked pose of the patient tracker is directly low-pass filtered (filter F2). As this measurement is not part of the closed control loop, this filter F2 can be set independently, i.e. to a higher cut-off frequency than the filter F1 integrated in the closed control loop.

Both filtered transformation matrices are then combined to the pose of the base of the robot in the coordinate system of the patient and then inverted to $^BT_P$.

The target tool position is given in the patient's coordinate system as $^PT_T$, target. With the synergistic control approach with hands-on control, this desired tool position can be calculated from the admittance control with the virtual fixtures applied. Alternatively, the desired tool position could be calculated from a trajectory handler during an automated working (e.g. milling) process by the tool.

This results in the desired tool pose in the coordinate system of the base of the tooling robot $^BT_{T, cmd}$ which can then be commanded to the actuation unit of the tooling robot.

Thanks to the kinematic model of the tooling robot, the joint positions $q_{cmd}$ are calculated and sent to the motion controllers.

The dashed line shows that the tracked tool position depends on the commanded motor position, thus additionally closing the control loop.

## REFERENCES

[1] Newman, W. S.: Stability and performance limits of interaction controllers. In: Journal of Dynamic Systems, Measurement and Control, Transactions of the ASME, 114 (1992), 4: 563-570

[2] Li, Zhijun; Huang, Bo; Ye, Zhifeng; Deng, Mingdi; Yang, Chenguang: Physical Human-Robot Interaction of a Robotic Exoskeleton By Admittance Control. In: IEEE Transactions on Industrial Electronics, 65 (2018), 12: 9614-9624

[3] Ott, Christian; Mukherjee, Ranjan; Nakamura, Yoshihiko: Unified impedance and admittance control. In: Proceedings - IEEE International Conference on Robotics and Automation (2010): 554-561

[4] Bowyer, Stuart A.; Davies, Brian L.; Rodriguez y Baena, Ferdinando: Active Constraints/Virtual Fixtures: A Survey. In: IEEE Transactions on Robotics, 30 (2014), 1: 138-157

## Claims

1. Robotic surgical system, comprising:

   - a robot (1A-1C) holding a surgical tool (3A-3C), the robot comprising a base (10), a flange (12) coupled to the surgical tool and an actuation unit (11) configured to move the flange (12) relative to the base (10);
   - a robotic carrier arm (2) comprising at least one joint (22A-22G), the carrier arm comprising a flange (23) removably connected to the base (10) of the robot (1A-1C), the carrier arm being configured to maintain position of the robot base (10) in a fixed joint configuration during operation of the robot (1A-1C);
   - a localization unit configured to determine in real time position of the surgical tool;

- a control unit configured to:

- determine the position of the surgical tool (3A-3C) based on data received from the localization unit;
- carry out a control loop for dynamically compensating elastic deformation of the carrier arm, the control loop comprising, based on a target position of the surgical tool, on a current joint position of the actuation unit, and on the position of the surgical tool, determining at least one command of the actuation unit to achieve the target position.

**2.** Robotic surgical system according to claim 1, wherein dynamically compensating the elastic deformation of the carrier arm further comprises determining the at least one command of the actuation unit based on a kinematic model of the actuation unit.

**3.** Robotic surgical system according to claim 1 or claim 2, wherein dynamically compensating the elastic deformation of the carrier arm further comprises determining the at least one command of the actuation unit based on a dynamic model of the robotic carrier arm.

**4.** Robotic surgical system according to any one of claims 1 to 3 wherein the robotic carrier arm (2) has six or seven degrees of freedom.

**5.** Robotic surgical system according to any one of claims 1 to 4, wherein the robotic carrier arm (2) has a serial architecture.

**6.** Robotic surgical system according to any one of claims 1 to 5, wherein the control unit is further configured to:

- determine a current joint configuration of the robotic carrier arm (2);
- based on a model of the robotic carrier arm (2), determine at least the lowest resonant frequency of the carrier arm in the current joint configuration;

wherein the control loop comprises applying a filter (F1) configured to dampen movement of the actuation unit (11) of the robot (1A-1C) at least at the lowest resonant frequency of the robotic carrier arm (2).

**7.** Robotic surgical system according to claim 6, wherein the control loop comprises:

- determining a position of the surgical tool (3A-3C) in a first coordinate system;
- determining a position of the patient in a second coordinate system;
- based on a current joint configuration of the actuation unit (11) and a kinematic model of the actuation unit, determining a current position of the base (10) in a third coordinate system;
- based on the position of the surgical tool (3A-3C) in the first coordinate system and on the position of the base (10) in the third coordinate system, determining the current position of the base (10) in the first coordinate system;
- filtering at least the lowest resonance frequency of the carrier arm (2) out of the position of the base (10) in the first coordinate system;
- based on the position of the patient in the second coordinate system and on the filtered position of the base (10) in the first coordinate system, determining the position of the base (10) in a fourth coordinate system;
- based on a target position of the surgical tool (3A-3C) in the fourth coordinate system, determining a target position of the surgical tool in a fifth coordinate system;
- implementing the kinematic model of the actuation unit (11) to determine a command of the actuation unit to achieve the target position of the surgical tool (3A-3C).

**8.** Robotic surgical system according to any one of claims 6 to 7, wherein each joint (22A-22G) of the robotic carrier arm (2) comprises an encoder and the control unit is configured to determine the current joint configuration of the carrier arm based on data provided by each encoder.

**9.** Robotic surgical system according to any one of claims 1 to 8, wherein the robotic carrier arm (2) is configured to be moved manually by a user to bring the robot (1A-1C) to a target position.

**10.** Robotic surgical system according to claim 9, further comprising at least one sensor configured to determine an intention of the user when moving the robotic carrier arm (2) and the control unit is configured to command at least one motor of the robotic carrier arm based on measurements provided by said at least one sensor to reach a target pose of the flange (23) of the robotic carrier arm.

**11.** Robotic surgical system according to any one of claims 1 to 10, wherein the surgical tool (3A-3C) is configured to be moved manually by a user.

**12.** Robotic surgical system according to claim 11, further comprising at least one sensor configured to determine an intention of the user when moving the surgical tool (3A-3C), the control unit being configured to command the actuation unit (11) of the robot based on measurements provided by said sensor during operation of the surgical tool by the user.

13. Robotic surgical system according to any one of claims 9 to 12, wherein the control unit is configured to provide haptic guidance to the user when moving the robotic carrier arm (2) or the surgical tool (3A-3C).

14. Robotic surgical system according to any one of the preceding claims, wherein the localization unit comprises a camera and at least one array of markers (4) detectable by the camera is fixed to the surgical tool (3A-3C) to determine in real time the position of the surgical tool.

15. Robotic surgical system according to any one of the preceding claims, wherein the surgical tool (3A-3C) is a cutting tool, in particular a saw, a drilling tool, a burring tool, a reaming tool or a milling tool, a burning tool, or an implant holder.

FIGURE 1

FIGURE 2A                    FIGURE 2B              FIGURE 2C

FIGURE 3

Europäisches
Patentamt

European
Patent Office

Office européen
des brevets

# EUROPEAN SEARCH REPORT

Application Number

EP 22 17 8223

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/068845 A1 (SCHERS JONATHAN [FR] ET AL) 11 March 2021 (2021-03-11) | 1,2,4,5, 9,11,14, 15 | INV. A61B34/30 A61B34/00 |
| Y | * paragraphs 103, 104, 131-143, 166, 167, 178, 182-202, 224-258, 281-307, 363 * | 3,10,12, 13 | ADD. |
| A | | 6-8 | A61B34/20 A61B90/00 |
| Y | US 2020/261169 A1 (MILLER DANIEL N [US] ET AL) 20 August 2020 (2020-08-20) * paragraphs [0038], [0042], [0046] – [0048], [0050], [0058], [0064] – [0073], [0078] – [0085]; figures 1-5 * | 3,10,12, 13 | |
| X | US 2021/361295 A1 (LAVALLEE STÉPHANE [FR] ET AL) 25 November 2021 (2021-11-25)  * paragraphs [0153] – [0155], [0166] – [0169], [0174], [0175], [0187] – [0242]; figures 1-14 * | 1,2,4,5, 9,11,14, 15 | |
| A | US 2020/323540 A1 (KANG HYOSIG [US] ET AL) 15 October 2020 (2020-10-15) * paragraphs [0039] – [0043], [0047], [0048], [0058], [0060], [0072], [0075], [0080], [0099], [0111], [0112], [0116], [0122]; figures 1-19 * | 1-15 | TECHNICAL FIELDS SEARCHED (IPC)  A61B |
| A | US 2007/151389 A1 (PRISCO GIUSEPPE [US] ET AL) 5 July 2007 (2007-07-05) * paragraphs [0024] – [0026], [0059] – [0078]; figures 1-8 * | 1-15 | |

-/--

The present search report has been drawn up for all claims

| Place of search | Date of completion of the search | Examiner |
|---|---|---|
| The Hague | 24 November 2022 | Viidebaum, Mikk |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EUROPEAN SEARCH REPORT**

Application Number

EP 22 17 8223

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROOZING WESLEY ET AL: "Comparison of open-loop and closed-loop disturbance observers for series elastic actuators", 2016 IEEE/RSJ INTERNATIONAL CONFERENCE ON INTELLIGENT ROBOTS AND SYSTEMS (IROS), IEEE, 9 October 2016 (2016-10-09), pages 3842-3847, XP033011915, DOI: 10.1109/IROS.2016.7759565 [retrieved on 2016-11-28] * page 3842 – page 3847 * | 1-15 | |
| | | | TECHNICAL FIELDS SEARCHED (IPC) |

The present search report has been drawn up for all claims

| Place of search | Date of completion of the search | Examiner |
|---|---|---|
| The Hague | 24 November 2022 | Viidebaum, Mikk |

EPO FORM 1503 03.82 (P04C01)

page 2 of 2

## ANNEX TO THE EUROPEAN SEARCH REPORT
## ON EUROPEAN PATENT APPLICATION NO.

EP 22 17 8223

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2022

| Patent document cited in search report | | Publication date | Patent family member(s) | | Publication date |
|---|---|---|---|---|---|
| US 2021068845 | A1 | 11-03-2021 | AU | 2019270999 A1 | 29-10-2020 |
| | | | EP | 3569159 A1 | 20-11-2019 |
| | | | EP | 3793451 A1 | 24-03-2021 |
| | | | JP | 2021523787 A | 09-09-2021 |
| | | | US | 2021068845 A1 | 11-03-2021 |
| | | | WO | 2019219348 A1 | 21-11-2019 |
| US 2020261169 | A1 | 20-08-2020 | CN | 111315309 A | 19-06-2020 |
| | | | EP | 3684280 A2 | 29-07-2020 |
| | | | JP | 6880324 B2 | 02-06-2021 |
| | | | JP | 2021502173 A | 28-01-2021 |
| | | | KR | 20200052980 A | 15-05-2020 |
| | | | KR | 20210024229 A | 04-03-2021 |
| | | | US | 2020261169 A1 | 20-08-2020 |
| | | | US | 2021177535 A1 | 17-06-2021 |
| | | | WO | 2019094794 A2 | 16-05-2019 |
| US 2021361295 | A1 | 25-11-2021 | EP | 3551099 A1 | 16-10-2019 |
| | | | US | 2021361295 A1 | 25-11-2021 |
| | | | WO | 2018104439 A1 | 14-06-2018 |
| US 2020323540 | A1 | 15-10-2020 | AU | 2020272975 A1 | 09-12-2021 |
| | | | CN | 113811258 A | 17-12-2021 |
| | | | EP | 3952774 A1 | 16-02-2022 |
| | | | JP | 2022529147 A | 17-06-2022 |
| | | | KR | 20210154181 A | 20-12-2021 |
| | | | US | 2020323540 A1 | 15-10-2020 |
| | | | WO | 2020210621 A1 | 15-10-2020 |
| US 2007151389 | A1 | 05-07-2007 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NEWMAN, W. S.** Stability and performance limits of interaction controllers. *Journal of Dynamic Systems, Measurement and Control, Transactions of the ASME,* 1992, vol. 114 (4), 563-570
- **LI, ZHIJUN ; HUANG, BO ; YE, ZHIFENG ; DENG, MINGDI ; YANG, CHENGUANG.** Physical Human-Robot Interaction of a Robotic Exoskeleton By Admittance Control. *IEEE Transactions on Industrial Electronics,* 2018, vol. 65 (12), 9614-9624

- **OTT, CHRISTIAN ; MUKHERJEE, RANJAN ; NAKAMURA, YOSHIHIKO.** Unified impedance and admittance control. *IEEE International Conference on Robotics and Automation,* 2010, 554-561
- **BOWYER, STUART A. ; DAVIES, BRIAN L. ; RODRIGUEZ Y BAENA.** Ferdinando: Active Constraints/Virtual Fixtures: A Survey. *IEEE Transactions on Robotics,* 2014, vol. 30 (1), 138-157